# EUROPEAN PATENT APPLICATION

(11) **EP 4 378 309 A1**
(43) Date of publication of application: **05.06.2024**
(21) Application number: 23211953.7
(22) Date of filing: 24.11.2023
(51) Int. Cl.: A01N 1/02, A01P 1/00, A01P 3/00

(54) **STERILIZING BIOLOGICAL TISSUE WITH SUPERCRITICAL CARBON DIOXIDE**

(30) Priority: 25.11.2022 US 202263385000 P
(71) Applicant: Innovmedics LLC, Irvine, CA 92618 (US)
(72) Inventor: TAN, Bin, Irvine, CA 92604 (US)
(74) Representative: Carridge, Andrew Edward

(57) **Abstract**

A method of sterilizing biological tissue such as soft mammalian tissue that can be used to make bioprosthetic devices is disclosed. The method comprises contacting biological tissue with a bioburden reduction mixture, contacting biological tissue with a tissue drying mixture, and thereafter sterilizing the dry tissue with supercritical carbon dioxide. Bioprosthetic devices made using the methods disclosed herein include bioprosthetic heart valves and surgical tissue patches.

## Description

### CROSS REFERENCE TO RELATED APPLICATION(S)

The present application claims the benefit of the filing date of US Provisional Application No. 63/385,000, filed November 25, 2022, the entire disclosure of which is incorporated herein by reference.

### FIELD OF THE INVENTION

The present invention relates generally to sterilizing biological tissue and bioprosthetic devices made using biological tissue.

### BACKGROUND OF THE INVENTION

Many medical devices, such as tissue patches and bioprosthetic heart valves, are made using biological tissue, including, without limitation, mammalian soft tissue, and engineered tissue. To maintain the sterility of the device, such devices were commonly packaged and delivered in a fixative solution. However, increasingly such devices are packaged and delivered to end users in a form where the tissue is dry. Such dry tissue devices are often sterilized using ethylene oxide (EO) and then placed in a gas impermeable packaging before delivery to end users.

There are many disadvantages to using ethylene oxide for sterilization of medical devices made out of biological tissue. Ethylene oxide is a harmful carcinogenic gas, and certain by-products formed during EO sterilization are also harmful to patients. As a consequence, biological tissue medical devices sterilized using EO must undergo rigorous and, therefore, expensive testing to ensure that no harmful residual products remain on the device. Further, because EO is carcinogenic, sterilization facilities using EO must comply with stringent OSHA regulations and require use of expensive continuous emissions monitoring systems. Therefore, there is a need for alternative sterilization methods for medical devices made with biological tissue that are packaged in a dry form.

Sterilization with supercritical carbon dioxide (sCOz) is a known sterilization technique. For example, US patent no. 6,149,864 describes a method for sterilizing polymers for drug delivery and implantation by treating the material with supercritical fluid carbon dioxide at pressures in the range of 2000 to 3000 psi (140 to 210 bar) and temperatures between 30 and 45 °C for periods between 20 minutes and six hours, wherein sterilization is carried out in the presence of water to achieve effective sterilization. US patent no. 7,108,832 discloses a method for sterilizing substrates such as bone or glass fibers or test tubes, wherein the substrate is exposed to sCOz in the presence of additives such as trifluoroacetic acid and water. Such methods cannot be used to sterilize biological tissue with sCO₂ because exposing biological tissue to sCO₂ in the presence of water (or in a wet form) would freeze the tissue causing it to become brittle, irreversibly dehydrated, and unusable in a bioprosthetic device. US patent no. 7,008,591 disclosed treating biological tissue with sCO₂ but the patent does not provide any details on whether effective reduction of infectious agents is achieved. Further, exemplary embodiments are directed to using sCO₂ to sterilize soft tissue treated with an aqueous glutaraldehyde solution, without an intermediate tissue drying step prior to treatment with sCO₂. Thus, there is no indication that glutaraldehyde treated soft tissue undergoes any form of drying treatment prior to exposure to sCO₂. In the absence of such treatment, exposing aqueous glutaraldehyde treated soft tissue to sCO₂ would cause the tissue to freeze, irreversibly dehydrate, and make it unsuitable for use in a bioprosthetic device.

Therefore, there is a need for an improved method for sterilizing biological tissue that avoids the use of EO, and provides adequate sterilization without compromising the integrity and properties of the tissue being treated.

### SUMMARY OF THE INVENTION

Technology and methods are set forth herein for sterilizing biological tissue and for making bioprosthetic devices comprising biological tissue sterilized using the methods disclosed herein. Several of the details set forth below are provided to describe the following examples and methods in a manner sufficient to enable a person skilled in the relevant art to practice, make and use them. Several of the details and advantages described below, however, may not be necessary to practice certain examples and methods of the technology. Additionally, the technology may include other examples and methods that are within the scope of the claims but are not described in detail.

Embodiments of the instant technology provide systems and methods for sterilizing biological tissue, and making and sterilizing bioprosthetic devices comprising biological tissue.

Some embodiments of the instant technology are directed to a method of sterilizing a piece of soft mammalian tissue harvested from an animal cadaver, wherein the method comprises immersing the piece of soft mammalian tissue in a chemical fixative solution; contacting the piece of soft mammalian tissue with a first bioburden reduction mixture; contacting the piece of soft mammalian tissue with a capping solution; subjecting the piece of soft mammalian tissue to a tissue purification process; contacting the piece of soft mammalian tissue with a tissue drying mixture; and contacting the piece of soft mammalian tissue with supercritical carbon dioxide. In some embodiments, the soft mammalian tissue comprises bovine or porcine pericardium tissue. The step of immersing the piece of soft mammalian tissue in a chemical fixative solution in some embodiments comprises immersing the piece of soft mammalian tissue in an aqueous glutaraldehyde solution comprising less than 10% glutaraldehyde by weight, a surfactant, ethanol and formaldehyde, wherein the piece of soft mammalian tissue is immersed in said aqueous glutaraldehyde solution for a period of less than14 days. In other embodiments, the aqueous glutaraldehyde solution comprises 0.5% glutaraldehyde by weight and the pH of the aqueous glutaraldehyde solution is greater than 7, and the piece of soft mammalian tissue is immersed said aqueous glutaraldehyde solution for one day.

In some embodiments of the above method, the first bioburden reduction mixture comprises less than 2% by weight glutaraldehyde, less than 25% by weight ethanol and less than 2% by weight Tween 80, and the piece of soft mammalian tissue is contacted with said first bioburden reduction mixture for a time period of less than 2 days while the temperature of the first bioburden reduction mixture is maintained at less than 50°C. In yet other embodiments, the piece of soft mammalian tissue is contacted with the first bioburden reduction mixture for a time period of 2 hours while the temperature of the first bioburden reduction mixture is maintained at about 40 °C.

Further, in one embodiment of the aforementioned method, the capping solution comprises less than 20 mM ethanolamine, less than 120 mM sodium borohydride, and sufficient phosphate buffer to maintain the pH of the capping solution above 7, and the piece of soft mammalian tissue is contacted with said capping solution for about 4 hours while maintaining the temperature of the capping solution at about 30 °C.

In another embodiment of the above method, the tissue purification process comprises: immersing the soft mammalian tissue in an aqueous ethanol solution comprising at least 5% by weight ethanol for a period of greater than one minute; transferring the soft mammalian tissue from the aqueous ethanol solution to a chamber; and exposing the soft mammalian tissue in the chamber to supercritical carbon dioxide. In some embodiments, the piece of soft mammalian tissue is exposed to supercritical carbon dioxide at a pressure greater than about 1100 psi and a temperature greater than about 31.1 °C.

In some embodiments of the foregoing method, the tissue drying mixture comprises an aqueous mixture comprising less than 95% by weight glycerol, less than 70% by weight ethanol, and less than 5% water, and the piece of soft mammalian tissue is contacted with said tissue drying mixture for a time period of less than 24 hours and at a temperature of about 30 °C.

In yet another embodiment of the above method, the step of contacting the soft mammalian tissue with supercritical carbon dioxide comprises placing the piece of soft mammalian tissue in a gas permeable pouch, placing the gas permeable pouch containing the piece of soft mammalian tissue in a sterilization chamber, and injecting supercritical carbon dioxide into the sterilization chamber, and the gas permeable pouch containing the piece of soft mammalian tissue is kept in the sterilization chamber for a time period of between 60 - 180 minutes while the sterilization chamber containing supercritical carbon dioxide is maintained at a pressure between about 1100 - 3000 psi, and a temperature between about 31 - 55 °C. Because sCO₂ sterilization has minimal detrimental effect on biological tissue, biological tissue can be subjected to multiple cycles of sCO₂ sterilization, if needed.

In yet another embodiment of the above method, the step of contacting the soft mammalian tissue with supercritical carbon dioxide comprises placing the piece of soft mammalian tissue in a gas permeable pouch, placing the gas permeable pouch containing the piece of soft mammalian tissue in a sterilization chamber, wherein the sterilization chamber contains an aqueous solution of hydrogen peroxide having a concentration of 5% by weight hydrogen peroxide, and wherein the amount of aqueous hydrogen peroxide is 5 ml per liter of the volume of the sterilization chamber, and injecting supercritical carbon dioxide into the sterilization chamber, wherein the gas permeable pouch containing the piece of soft mammalian tissue is kept in the sterilization chamber for a time period of between 60 - 180 minutes while the sterilization chamber containing supercritical carbon dioxide is maintained at a pressure between about 1100 - 3000 psi, and a temperature between about 31- 55 °C.

Another embodiment of the technology disclosed herein is a method of sterilizing a piece of soft mammalian tissue harvested from an animal cadaver, wherein the method comprises immersing the piece of soft mammalian tissue in an aqueous glutaraldehyde solution comprising less than 2% glutaraldehyde by weight, a surfactant, and ethanol, wherein the piece of soft mammalian tissue is immersed in said aqueous glutaraldehyde solution for a period of less than14 days; contacting the piece of soft mammalian tissue with a bioburden reduction mixture comprising less than 2% by weight glutaraldehyde less than 25% by weight ethanol and less than 2% by weight Tween 80, wherein the piece of soft mammalian tissue is contacted with the bioburden reduction mixture for a time period of less than 2 days while the temperature of the bioburden reduction mixture is maintained at less than 50 °C; contacting the piece of soft mammalian tissue with a tissue drying mixture comprising an aqueous mixture comprising less than 95% by weight glycerol, less than 70% by weight ethanol, and less than 5%, water wherein the piece of soft mammalian tissue is contacted with the tissue drying mixture for a time period of less than 24 hours and at a temperature of about 30 °C; placing the piece of soft mammalian tissue in a gas permeable pouch; placing the gas permeable pouch containing the piece of soft mammalian tissue in a sterilization chamber; and injecting supercritical carbon dioxide into the sterilization chamber, wherein the gas permeable pouch containing the piece of soft mammalian tissue is kept in the sterilization chamber for a time period of between 60 - 180 minutes while the sterilization chamber containing supercritical carbon dioxide is maintained at a pressure between about 1100 - 3000 psi, and a temperature between about 31 - 55 °C. In one embodiment of this method, prior to the step of contacting the piece of soft mammalian tissue with the tissue drying mixture, the piece of soft mammalian tissue is contacted with normal saline to remove the residual glutaraldehyde and other chemicals followed by capping the free aldehyde group with an amino acid solution such as 0.1 M glycine solution in phosphate buffered saline at pH of 7.35 to 7.45. In another embodiment of the foregoing method, after the step of anti-calcification treatment, the piece of soft mammalian tissue undergoes a tissue purification treatment comprising: immersing the soft mammalian tissue in an aqueous ethanol solution comprising at least 5% by weight ethanol for a period of greater than one minute; transferring the soft mammalian tissue from the aqueous ethanol solution to a chamber; and exposing the soft mammalian tissue in the chamber to supercritical carbon dioxide. In some embodiments, the piece of soft mammalian tissue is exposed to supercritical carbon dioxide at a pressure greater than about 1100 psi and a temperature greater than about 31.1 °C.

Another embodiment of the instant technology is a method of sterilizing a piece of soft mammalian tissue harvested from an animal cadaver, wherein the method comprises contacting the piece of soft mammalian tissue with a tissue drying mixture comprising an-aqueous mixture comprising less than 95% by weight glycerol, less than 70% by weight ethanol,, and less than 5% water, wherein the piece of soft mammalian tissue is contacted with the tissue drying mixture for a time period of less than 24 hours and at a temperature of about 30 °C; placing the piece of soft mammalian tissue in a gas permeable pouch; placing the gas permeable pouch containing the piece of soft mammalian tissue in a sterilization chamber; and injecting supercritical carbon dioxide into the sterilization chamber, wherein the gas permeable pouch containing the piece of soft mammalian tissue is kept in the sterilization chamber for a time period of between 60 - 180 minutes while the sterilization chamber containing supercritical carbon dioxide is maintained at a pressure between about 1100 - 3000 psi, and a temperature between about 31 - 55 °C. In one embodiment of the foregoing method, prior to the step of contacting the piece of soft mammalian tissue with the tissue drying mixture, the piece of soft mammalian tissue undergoes anti-calcification treatment comprising washing the soft mammalian tissue with normal saline, and contacting the soft mammalian with a solution comprising 0.1 M glycine and phosphate buffered saline at pH of 7.35 to 7.45. In another embodiment of the above method,

In yet another aspect, embodiments of the present technology provide methods of making bioprosthetic devices comprising soft mammalian tissue such that the device can be packaged in a sterile and dry form for delivery to end users. For example, in one embodiment, the technology disclosed herein provides a method of manufacturing a tissue surgical patch comprising a piece of soft mammalian tissue harvested from an animal cadaver, wherein the method comprises contacting the piece of soft mammalian tissue with a tissue drying mixture comprising an aqueous mixture comprising less than 95% by weight glycerol, less than 70% by weight ethanol, and less than 5% water, wherein the piece of soft mammalian tissue is contacted with the tissue drying mixture for a time period of less than 24 hours and at a temperature of about 30 °C; cutting the tissue surgical patch out of the piece of soft mammalian tissue; placing the tissue surgical patch in a gas permeable pouch; placing the gas permeable pouch containing the tissue surgical patch in a sterilization chamber; and injecting supercritical carbon dioxide into the sterilization chamber, wherein the gas permeable pouch containing the tissue surgical patch is kept in the sterilization chamber for a time period of between 60 - 180 minutes while the sterilization chamber containing supercritical carbon dioxide is maintained at a pressure between about 1100 - 3000 psi, and a temperature between about 31- 55 °C. In some embodiments of the foregoing method, the piece of soft mammalian tissue comprises bovine or porcine pericardial tissue.

Another embodiment is a variation of the foregoing method to make a bioprosthetic tissue heart valve in a sterile and dry form for packaging and delivery to end users. In one embodiment, such a method of making a bioprosthetic heart valve comprises cutting heart valve leaflets out of the piece of bovine pericardium tissue; assembling the bioprosthetic heart valve using said heart valve leaflets and other structural components of a bioprosthetic heart valve; contacting the bioprosthetic heart valve with a tissue drying mixture comprising an aqueous mixture comprising less than 95% by weight glycerol, less than 70% by weight ethanol, and less than 5% water, wherein the bioprosthetic heart valve is contacted with the tissue drying mixture for a time period of less than 24 hours and at a temperature of about 30 °C; placing the bioprosthetic heart valve in a gas permeable pouch; placing the gas permeable pouch containing the bioprosthetic heart valve in a sterilization chamber; and injecting supercritical carbon dioxide into the sterilization chamber, wherein the gas permeable pouch containing the bioprosthetic heart valve is kept in the sterilization chamber for a time period of between 60 - 180 minutes while the sterilization chamber containing supercritical carbon dioxide is maintained at a pressure between about 1100 - 3000 psi, and a temperature between about 31 - 55 °C.

### BRIEF DESCRIPTION OF THE DRAWINGS

Certain aspects of the present disclosure can be understood with reference to the following drawings:
FIG. 1 compares the denaturation temperature of dried+ sCO₂ sterilized bovine pericardial tissue with the denaturation temperatures of three other types of bovine pericardial tissue.
FIG. 2 compares the denaturation temperature of dried+ sCO₂ sterilized porcine pericardial tissue with the denaturation temperatures of three other types of porcine pericardial tissue.
FIG. 3 compares the yield stress of dried+ sCO₂ sterilized bovine pericardial tissue with the yield stresses of two other types of bovine pericardial tissue.
FIG. 4 is a temperature-pressure phase diagram of carbon dioxide.
FIG. 5 shows microbial log reduction at various sterilization pressures for a bioindicator strip sterilized with sCO₂.
FIG. 6 shows microbial log reduction at various sterilization temperatures for a bioindicator strip sterilized with sCO₂.
FIG. 7 shows microbial log reduction at various sterilization cycle durations for a bioindicator strip sterilized with sCO₂.
FIG. 8 shows microbial log reduction at various number of sterilization cycles for a bioindicator strip sterilized with sCO₂.
FIG. 9 shows a process flow chart for an embodiment of the methods disclosed herein for manufacturing a surgical patch comprising biological tissue.
FIG. 10 shows a process flow chart for an embodiment of the methods disclosed herein for manufacturing a bioprosthetic heart valve comprising biological tissue.

### DETAILED DESCRIPTION OF THE INVENTION

Specific details of several embodiments of methods for sterilizing biological tissue and bioprosthetic devices, such as surgical tissue patches and bioprosthetic heart valves, comprising biological tissue sterilized using the methods herein, and methods of making such devices are described below. Although many of the embodiments are described below with respect to surgical patches and bioprosthetic heart valves, other applications and other embodiments in addition to those described herein are within the scope of the technology. Additionally, several other embodiments of the technology can have different configurations, components, or procedures than those described herein. A person of ordinary skill in the art, therefore, will accordingly understand that the technology can have other embodiments with additional elements, or the technology can have other embodiments without several of the features shown and described below.

The headings provided herein are for convenience only.

### Biological Tissue

The present invention provides an improved bioprosthetic tissue treatment process for packaging and storing biological tissue, and bioprosthetic devices that are made using biological tissue. The tissues with which the present method is practiced include substantially any biological-tissue that is useful in preparing a prosthetic device having a biological component thereto such as soft mammalian tissue or engineered tissue. Thus, biological tissue includes, without limitation, soft mammalian tissue such as bovine pericardium and porcine tissue which are commonly used in bioprosthetic heart valves, blood vessels, skin, dura mater, pericardium, peritoneum, small intestinal submucosa ("SIS tissue"), tissue heart valves, ligaments and tendons. For example, in one embodiment, the tissue is derived from an organ. In another embodiment, the soft-tissue is selected from nerve tissue, glandular tissue (e.g., lymphatic tissue), respiratory tissue, digestive tissue, urinary tract tissue, sensory tissue (e.g., cornea, lens, etc.), and reproductive tissue. In some embodiments, the tissue is selected from muscle tissue, adipose tissue, epithelial tissue and endothelial tissue. In particular embodiments, the tissue is selected from myocardial tissue and vascular tissue. In other embodiments, the biological tissue comprises engineered tissue. Engineered tissue comprises (i) a three-dimensional supporting structure, the so-called scaffold, and (ii) cellular components that bind to the scaffold. Scaffolds can comprise synthetic materials including, without limitation, polyglycolic acid, polylactic acid, polyhydroxyalkanoates and poly-4-hydroxybutyrate. Alternatively, the scaffold can comprise biologically derived extracellular matrix (ECM) obtained by decellularizing allogenic or xenogenic tissue. The decellularized ECM can be seeded with, for example, fibroblasts and endothelial cells derived from autologous adipose tissue-derived stem cells. The bioprosthetic device comprising engineered tissue may be repopulated by autologous cells post-implantation.

### Biological Tissue Bioprosthetic Devices

In accordance with some embodiments of the technology disclosed herein, bioprosthetic devices are comprised of biological tissue treated with the methods disclosed herein. If the biological tissue is to be attached to a bioprosthetic device for implantation, the tissue can be treated with the methods disclosed herein prior to its attachment or after its attachment to the bioprosthetic device. For example, bovine or porcine pericardium can be treated with the methods disclosed herein (i) prior to the time it is formed into a heart valve, vascular graft, stent covering, or pericardial patch or (ii) after the bovine or porcine pericardium is formed into a heart valve, vascular graft, stent covering, or pericardial patch. Furthermore, when the methods disclosed herein comprise multiple steps, one or more steps may be performed on the biological tissue prior to the time the tissue is formed into a bioprosthetic device, e.g., heart valve, vascular graft, stent covering, or pericardial patch, and the remaining steps may be performed on the tissue after the bovine or porcine pericardium is formed into the bioprosthetic device.

An exemplary embodiment of a bioprosthetic device that can be made using the methods disclosed herein is a tissue patch made using biological tissue. Such tissue patches can be used in a variety of surgical procedures. Thus, in various embodiments, uses of tissue patches prepared in accordance with the methods disclosed herein include, without limitation, (i) use of the tissue patch as a dura substitute for the closure of dura mater during neuro surgery, (ii) use of the tissue patch as a prosthesis for pericardial closure and soft tissue deficiencies which include: defects of the abdominal and thoracic wall, hernias (diaphragmatic, femoral, incisional, inguinal, lumbar, paracolostamy, scrotal and umbilical), and intercardiac and great vessel repair, (iii) use of the tissue patch to assist in closure following open heart surgery, (iv) use of the tissue patch for suture line reinforcement, and (v) use of the tissue patch for peripheral vascular reconstruction including the carotid, renal, iliac, femoral, profunda and tibial blood vessels and arteriovenous access revisions. In some embodiments, tissue patches for above applications are made from bovine pericardium. In various embodiments, in size the tissue patch can be 2×2 cm, 2×8 cm, 4×4 cm or 5×8 cm.

An embodiment of the technology disclosed herein is also directed to a bioprosthetic tissue heart valve comprising biological tissue, such as one in which valve leaflets are made with bovine pericardium, or one in which a whole porcine heart valve is used, treated in accordance with methods disclosed herein. The biological tissue can be processed according to the described methods, at various steps of the method, prior to or following its attachment to the other structural elements of the bioprosthetic heart valve. A stented bioprosthetic heart valve has multiple biological tissue leaflets joined together at a periphery of the valve at valve commissures that are generally axially aligned and evenly disposed about a valve axis. The valve commissures are each disposed between adjacent curvilinear valve cusps along the periphery of the valve. The bioprosthetic heart valve may further include a holder that comprises a plurality of cusp supports arranged around an axis to contact the heart valve generally along the valve cusps.

Another embodiment of the technology disclosed herein is a composite valved conduit that comprises a bioprosthetic valve attached to a conduit graft, wherein the bioprosthetic valve comprises biological tissue that has been treated by the methods disclosed herein. The conduit graft is impregnated with bioresorbable materials such as collagen or gelatin. The bioprosthetic valve, either a stented valve comprising leaflets made with bovine or porcine pericardium or a whole porcine valve, is attached to the conduit graft either using sutures or a snap fit connection.

### Fixation of Soft Tissue

It is known that the tensile properties and antigenic reaction of natural tissue that is to be used in a bioprosthesis may be improved if the tissue is chemically fixed (or tanned). The techniques used for chemical fixation of biological tissues typically involve the exposure of the biological tissue to one or more chemical fixatives (i.e., tanning agents) that form cross-linkages between the polypeptide chains within a given collagen molecule (i.e., intramolecular crosslinkages), or between adjacent collagen molecules (i.e., intermolecular crosslinkages). Examples of chemical fixative agents that have been utilized to crosslink collagenous biological tissues include: formaldehyde, glutaraldehyde, dialdehyde starch, genipin, 1-ethyl-3-(3-dimethylaminopropyl) carbodiimide (EDC), hexamethylene diisocyanate and certain polyepoxy compounds. Of the various chemical fixatives available, glutaraldehyde has been the most widely used since the discovery of its anti-immunogenic and antidegenerative effects. In addition, glutaraldehyde is one of the most efficient sterilization agents. Glutaraldehyde is used as a fixative and sterilant for many commercially available bioprosthetic products, such as porcine bioprosthetic heart valves or bovine pericardial heart valves.

Chemical fixation of biological tissue can be accomplished by using a 0.1% to 10% glutaraldehyde solution as the tanning agent. In one embodiment, chemical fixation is carried out by immersing the tissue in a solution of 0.5% by weight glutaraldehyde buffered to a pH of approximately 7.4 by a suitable buffer such as a phosphate buffer, for 1-14 days at ambient temperature. In order to enhance fixation or sterilization other chemical compounds such as surfactants (e.g. Tween^{®} 80) and/or ethanol and/or formaldehyde can be added to the glutaraldehyde. It will be appreciated, however, that various other fixatives may be used, such as aldehydes (e.g., formaldehyde, glutaraldehyde, dialdehyde starch) or polyglycidyl ethers (e.g., Denacol 810), or heterologous bifunctional or multifunctional crosslinkers.

### Bioburden Reduction Treatment

After the mammalian tissue has been chemically fixed, in one embodiment it is subjected to one or more bioburden reduction treatments. Below are exemplary bioburden reduction treatments which are commonly used but other bioburden reduction treatments known to those of ordinary skill in the art are also contemplated by the technology herein.

First Bioburden Reduction Treatment: The fixed tissue is contacted with a mixture containing i) a crosslinking agent, such as formaldehyde or glutaraldehyde ii) a denaturing agent, such as ethanol and iii) a surfactant, such as commercially available Tween^{®} 80 or Tween 20 surfactant. In one embodiment, the crosslinking agent, denaturing agent, and surfactant mixture has the following composition: glutaraldehyde 2.0±0.2% by weight, ethanol 25.0±2.5% by weight, and Tween^{®} 80 1.2±0.2% by weight. The tissue is immersed in this mixture, in one embodiment, for 2 hours to 7 days, and in one particular embodiment for about 2 hours. During this immersion period, in one embodiment, the mixture is maintained at a temperature of 4-50 °C, and in a particular embodiment at about 20-40 °C.

In various embodiments, the crosslinking agent for the aforesaid mixture can be selected from the group consisting of the following compounds or mixtures thereof: formaldehyde, glutaraldehyde, paraformaldehyde, glyceraldehyde, glyoxal acetaldehyde, acrolein, any of the various Denacols and their individual reactive species, including mono, di, tri, and multi-functionalized epoxides, carbodiimides, mixed multifunctional molecules (e.g. aldehyde-epoxide combination), and mixtures thereof. In some embodiments, the denaturing agent for the aforesaid mixture can be selected from the group consisting of the following compounds or mixtures thereof: alcohols/solvents (e.g., ethanol, isopropyl alcohol), acidified ethers (e.g., sulfuric acid/ether mixture), acetone, ethers of small alkyl size (methyl, ethyl, etc. but probably not beyond butyl), ketones (e.g., methyl ethyl ketone (MEK)), commercial solvent systems (e.g., Genesolve^{™} , glycerol ethylene glycol, polyethylene glycol, low molecular weight carbowax, chaotropic agents (e.g., urea, guanidine hydrochloride, guanidine thiocyanate, potassium iodide), and high concentration salt solutions (e.g., lithium chloride, sodium chloride, cesium chloride). In some embodiments, the surfactant for the aforesaid mixture can be selected from the group consisting of the following compounds or mixtures thereof: anionic surfactants (e.g., esters of lauric acid, including but not limited to sodium laurel sulfate (also called sodium dodecyl sulfate)), alkyl sulfonic acid salts (e.g., 1-decanesulfonic acid sodium salt), non-ionic compounds (e.g., compounds based on the polyoxyethylene ether structures, including Triton X-IOO, 114, 405, N-101 (available commercially), pluronic and tetronic surfactants (available commercially), and alkylated phenoxypolyethoxy alcohols (e.g., NP40, Nonidet P40, Igepal, CA630, hydrolyzedlfunctionalized animal and plant compounds including Tween^{®} 80, Tween^{®} 20, octyl-derivatives, octyl b-glucoside, octyl bthioglucopyranoside, deoxycholate and derivatives thereof, zwitterionic compounds, 3-([cholamidopropyl]-dimethyl amino)-1-propanesulfonate (CHAPS), 3-([cholamidopropyl]-dimethyl amino)-2-hydroxy-1-propanesulfonat-e).

Second Bioburden Reduction Treatment: In some embodiments, the tissue can be subjected to a second bioburden reduction treatment, which comprises contacting the tissue with essentially the same mixture as in the first bioburden reduction treatment for a period of 2 hours to 10 days at a temperature of 35-40°C. In one embodiment, the second bioburden reduction treatment is performed by contacting the tissue with the same mixture as in the first bioburden reduction treatment at a temperature of 40°C, for a period of about 16 hours.

### Anti-Calcification Treatment

In between the two bioburden reduction treatments, the fixed tissue can be treated to improve its anti-calcification properties. As is known to a person of ordinary skill in the art, chemical fixation of biological tissue, while improving its anti-immunogenic properties makes the fixed tissue vulnerable to *in vivo* calcification after the tissue (or bioprosthetic device comprised of the tissue) is implanted in a human subject. Over time calcification will degrade the properties of the tissue and its functionality (or that of the bioprosthetic device comprised of the tissue).

In some embodiments, the fixed biological tissue (or bioprosthetic device comprised of the tissue) is subjected to anti-calcification treatment that comprises contacting the fixed mammalian tissue (or bioprosthetic device comprised of the tissue) with a solution comprising a capping agent and an anti-oxidant. The capping agent is a compound selected from the group consisting of amines (e.g., alkyl amines, amino alcohols, ethanolamine), amino acids (e.g., lysine, hydroxylysine), amino sulfonates (e.g., taurine, amino sulfates, dextran sulfate, chondroitin sulfate), hydrophilic multifunctional polymer (e.g., polyvinyl alcohol, polyethyleneimine), hydrophobic multifunctional polymer, α-dicarbonyls (e.g., methylglyoxal, 3-deoxyglucosone, glyoxal), hydrazides (e.g., adipic hydrazide), N,N-disuccinimidyl carbonate, carbodiimides (e.g., 1-ethyl-3-[3-dimethylaminopropyl]carbodiimide hydrochloride (EDC), N-cyclohexyl-N' -(2-morpholinoethyl)carbodiimide (CMC), 1,3-dicyclohexyl carbodiimide (DCC), 2-chloro-1-methylpyridinium iodide (CMPI)), an antibiotic, a cell recruiting agent, a hemocompatibility agent, heparin, an anti-inflammatory agent, an antiproliferative agent, an immunogenic suppressing agent, a reducing agent, sodium cyanoborohydride, sodium borohydride, sodium bisulfite+acetylacetone, formic acid+formaldehyde, and mono-, di- or polyepoxy alkanes, and mixtures thereof. The anti-oxidant is selected from the group consisting of a water soluble antioxidant (such as ascorbic acid), a fat soluble antioxidant (such as tocopherols), a carbohydrate (such as fructose, sucrose, or mannitol) a hindered phenol (such as butylated hydroxytoluene (BHT)), a hindered amine light stabilizer (HALS) (such as p-phenylamine diamine, trimethyl dihydrodquinoline, or alkylated diphenyl amines), a phosphite/phosphonite (such as triphenyl phosphine), a thioester (such as a thiocinnamate), and mixtures thereof.

In one embodiment, the fixed biological tissue (or bioprosthetic device comprised of the tissue) is rinsed in an ethanol/saline solution (20% ethanol) to remove any excess glutaraldehyde adhering to the tissue. The fixed mammalian tissue (or bioprosthetic device comprised of the tissue) is then contacted with normal saline to remove the residual glutaraldehyde and other chemicals followed by capping the free aldehyde group with amino acid solution such as 0.1 M glycine solution in phosphate buffered saline at pH 7.35 to 7.45. Tissue Purification Treatment

In some embodiments, the biological tissue (or bioprosthetic device comprised of the tissue) is subjected to a tissue purification treatment which comprises first immersing the biological tissue (or bioprosthetic device comprised of the tissue) in a aqueous ethanol solution at room temperature for less than 180 minutes, and transferring the biological tissue (or bioprosthetic device comprised of the tissue) soaked in ethanol solution quickly to a chamber connected to a source of supercritical carbon dioxide (sCO₂). The term "quickly" should be understood to mean that the transfer of the biological tissue (or bioprosthetic device comprised of the tissue) to the chamber is done with sufficient speed so that very little ethanol evaporates from the tissue during the transfer. In various embodiments, the transfer takes place in less than 10 minutes. Supercritical CO₂ (that is, CO₂ at a minimum pressure and temperature of 1099 psi and 31.1 °C, respectively) is then injected into the chamber and the chamber is maintained under supercritical conditions for 5 - 180 minutes. Carbon dioxide is then vented from the chamber and the purified biological tissue (or bioprosthetic device comprised of the tissue) is removed from the chamber. The ethanol concentration of the aqueous ethanol solution in which the biological tissue (or bioprosthetic device comprised of the tissue) is immersed in various embodiments is (i) 5-95 weight percent ethanol, (ii) 10-85 weight percent ethanol, (iii) 20-75 weight percent ethanol, (iv) 30-65 weight percent ethanol, or (v) 40-55 weight percent ethanol. In certain embodiments, the second bioburden reduction treatment set forth above need not be performed if the biological tissue (or bioprosthetic device comprised of the tissue) is subjected to the tissue purification treatment set forth herein.

### Tissue Drying Treatment

Traditionally, bioprosthetic devices comprising biological tissue were packaged and delivered to end users (hospitals, medical personnel, such as surgeons) in a wet form, for example, immersed in a fixative solution such as a glutaraldehyde solution to maintain the tissue in a hydrated and sterile state. However, this approach required the end user to spend considerable time rinsing the bioprosthetic device to remove the fixative solution. Accordingly, recently, tissue drying treatment processes that allow for dry packaging and delivery of bioprosthetic devices have been developed, and now commonly bioprosthetic devices comprising biological tissue are packaged and delivered to end users (hospitals, medical personnel, such as surgeons) in a dry form.

Glutaraldehyde fixed biological tissue, such as bovine or porcine pericardial tissue, that has not been subjected to a drying treatment would comprise about 75±5% by weight water. This water comprises water molecules that are an essential part of the structure of biological tissue, and excess free water molecules. The excess free water can be removed from biological tissue either (i) replacing it with less volatile chemicals, such as glycerol, which enable the tissue to be stored in a dry condition because chemicals such as glycerol do not readily evaporate when exposed or stored in air for a substantial period of time, or (ii) lyophilizing the tissue. Treating the tissue with a chemical such as glycerol will sufficiently dry the tissue such that when the tissue is sterilized using supercritical carbon dioxide, the tissue will not freeze, which must be avoided because if the tissue freezes it will become brittle and be irreversibly dehydrated. The water content of sufficiently dry biological tissue will be substantially less than that of fixed biological tissue which has not been dried.

In one embodiment, the treatment process for drying tissue involves treating the tissue with a polyhydric alcohol, such as glycerol. As used herein, the term "polyhydric alcohol" refers to an organic molecule that contains a plurality of carbon atoms and two or more hydroxyl groups, wherein the hydroxyl groups are attached to carbon atoms. Examples include glycerol, ethylene glycol, polyethylene glycols, propylene glycol, butylene glycol and derivatives of glycerol. Needless to say, mixtures of two or more polyhydric alcohols, such as a mixture of glycerol and propylene glycol, can be used in the processes described herein.

In a particular embodiment, the tissue is contacted with a nonaqueous mixture of a polyhydric alcohol and one or more C₁-C₃ alcohols. The C₁-C₃ alcohol is selected from the group consisting of methanol, ethanol, isopropanol, n-propanol, and mixtures thereof.

The tissue drying treatment process can be performed at various points in the methods of tissue treatment disclosed herein. For example, the tissue drying treatment process can be performed (a) after the tissue (or the bioprosthetic device comprised of the tissue) has been treated with a chemical fixative such as glutaraldehyde, or (b) after the chemically fixed tissue (or the bioprosthetic device comprised of the tissue) has been subjected to a first bioburden reduction treatment, or (c) after the chemically fixed tissue (or the bioprosthetic device comprised of the tissue) has been subjected to a calcification mitigation treatment, or (d) after the chemically fixed tissue (or the bioprosthetic device comprised of the tissue) has been subjected to a first bioburden reduction treatment, a calcification mitigation treatment, and a second bioburden reduction treatment. The tissue drying treatment process in one embodiment comprises contacting fixed (for example, with glutaraldehyde) tissue (before or after the tissue is incorporated in the bioprosthetic device) with an aqueous mixture comprising glycerol and ethanol. The tissue for example, can be placed in a bath containing such an aqueous mixture. In some embodiments, the aqueous mixture comprises 30-95% glycerol, 5 - 70% ethanol, and less than 5% water. In one particular embodiment, the aqueous mixture comprises 80% glycerol and 19% ethanol, and 1% water. The concentrations of glycerol and ethanol in the mixture are varied depending on the type of tissue or bioprosthetic device being treated. The type of tissue or bioprosthetic device also determines the time of contact of the tissue with the aqueous mixture. In some embodiments, the time of contact is between 1-24 hours. For example, the time of contact can be 2 hours, 4 hours, 8 hours, 12 hours, 16 hours, 20 hours or 24 hours. In one embodiment, the biological tissue (or the bioprosthetic device that comprises biological tissue, such as a bioprosthetic heart valve comprising bovine or porcine pericardium tissue) after chemical fixation treatment is contacted with an aqueous solution containing 80% by weight glycerol, 19%by weight ethanol, and 1% water for one hour at room temperature. After contact for one hour, the biological tissue (or the bioprosthetic device) is removed from the glycerol/ethanol solution and exposed to ambient air or an inert environment, e.g., nitrogen, at standard room temperature and humidity so as not to adversely affect tissue properties (typically, at a temperature from about 15 °C to about 25 °C, and relative humidity preferably less than about 50%). In some embodiments, the exposure is performed in a clean room or in a clean hood at ambient room conditions for about 1-4 hours. This exposure allows the excess glycerol/ethanol mixture to drip off/evaporate from the biological tissue (or bioprosthetic device), resulting in substantially dry biological tissue (or bioprosthetic device).

An alternative method for drying biological tissue is to lyophilize the tissue. Lyophilization is well-known to those of skill in the art but generally it comprises freeze drying the tissue using a process wherein water is removed from tissue after it is frozen and placed under a vacuum, allowing the ice that froze out of the tissue to change directly from solid to vapor without passing through a liquid phase

### Supercritical Carbon Dioxide Sterilization

A series of studies were conducted to evaluate the feasibility and efficacy of using supercritical fluid carbon dioxide (sCO₂) as a sterilization method to sterilize implantable medical devices, particularly bioprosthetic heart valves and biological tissue products such as surgical tissue patches. Although, sCO₂ sterilization has been studied as sterilization method for polymer and other biological material-derived medical devices such as artificial bones, no studies for using sCO₂ to sterilize biological tissue, such as pericardium-derived medical devices such as pericardial patch and bioprosthetic heart valves, have been reported. Therefore, a number of experiments were conducted to study the feasibility and efficacy of using sCO₂ sterilization for sterilizing biological tissue and devices comprising such tissue.

Effect of sCO₂ sterilization on tissue crosslinking: Implantable medical device comprising soft mammalian tissue (tissue patches or bioprosthetic heart valves) are preserved or fixed with glutaraldehyde to crosslink the side chains of amine-group of an amino acid to the carboxylic group of the adjacent amino acid. Tissue crosslinking is critically important to tissue performance, durability, and biocompatibility and, therefore, any changes in tissue processing, packaging/storage, and sterilization that may impact the tissue crosslinking must be assessed.

The effect of any process on tissue crosslinking can be studied using differential scanning calorimetry (DSC) to determine the denaturation temperature of the tissue, which is useful measure of the degree of crosslinking of the tissue. If a process does not change the denaturation temperature of a tissue by more than 3 °C, it can be concluded that the process has an negligible effect on the crosslinking of the tissue.

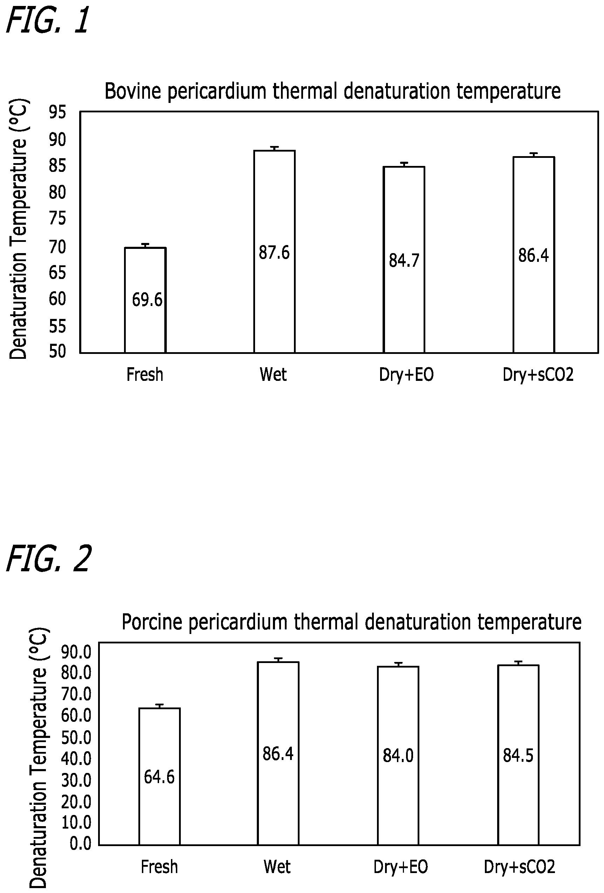

In this study, DSC was used to determine the denaturation temperature of four types of tissue: (a) fresh or unfixed bovine and porcine pericardial tissue (Fresh), (b) glutaraldehyde fixed wet bovine and porcine pericardial tissue (Wet), (c) glutaraldehyde fixed bovine and porcine pericardial tissue, subsequently dried using glycerolization and then sterilized using ethylene oxide (Dry+EO), and (d) glutaraldehyde fixed bovine and porcine pericardial tissue, subsequently dried using glycerolization and then sterilized using sCO₂ (Dry+sCO₂). The denaturation temperature results for these four tissue forms for bovine pericardial tissue and porcine pericardial tissue are shown in FIGS. 1 and 2, respectively. As can be seen from the figures, in the case of bovine pericardium tissue, the denaturation temperature of Wet tissue was 87.6 °C and of Dry+sCO₂ tissue was 86.4 °C, and in the case of porcine pericardium tissue, the denaturation temperature of Wet tissue was 86.4 °C and of Dry+sCO₂ tissue was 84.5 °C. Thus, in both cases the denaturation temperature of sCO₂ sterilized tissue is within 2 °C of the Wet tissue. Therefore, it can be concluded that sCO₂ sterilization does not affect the crosslinking of either bovine or porcine pericardial tissue.

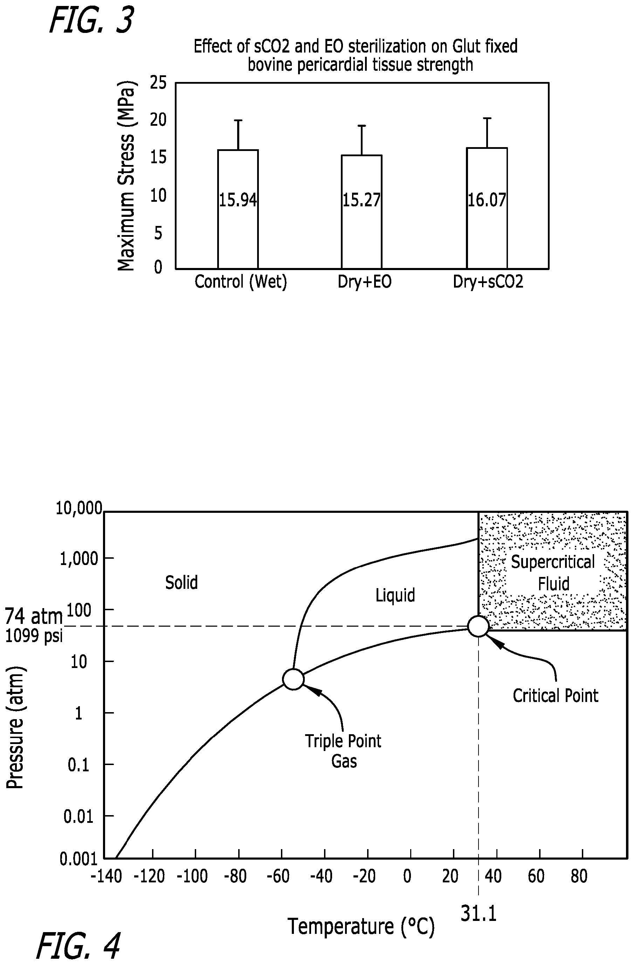

Effect of sCO₂ sterilization on tissue mechanical properties: In addition to tissue crosslinking, another important consideration is the effect of sCO₂ sterilization on the tissue's mechanical properties which dictate the function and durability of the tissue-derived devices. To determine the effect of sCO₂ sterilization on tissue mechanical properties, the yield stress was measured for three types of tissue: (a) glutaraldehyde fixed wet bovine pericardial tissue (Wet), (b) glutaraldehyde fixed bovine pericardial tissue, subsequently dried using glycerolization and then sterilized using ethylene oxide (Dry+EO), and (c) glutaraldehyde fixed bovine pericardial tissue, subsequently dried using glycerolization and then sterilized using sCO₂ (Dry+sCO₂). The yield stress results are presented in FIG. 3, which shows that sCO₂ sterilization has no appreciable detrimental effect on the pericardial tissue yield stress and, therefore, its mechanical properties.

The effect of sterilization parameter on sCO₂ sterilization efficacy: As shown in FIG. 4, the minimal pressure and temperature for CO₂ to achieve supercritical state is 1099 psi and 31.1 °C. A number of experiments were carried out to determine the effect of sterilization pressure, sterilization temperature, sterilization cycle duration and the number of sterilization cycles on the efficacy of sCO₂ sterilization. These experiments were conducted with a biological indicator (Steris, Cat# NA004)), which is a strip loaded with live bacteria. After the sCO₂ sterilization, the bioindicators were tested for bacterium growth. The bacterium growth is estimated or quantified as the number of colony forming unit per test sample (cfu/sample). An unsterilized bioindicator strip was used as control. The log reduction was calculated based on the difference of cfu numbers between the control and the sterilized sample.

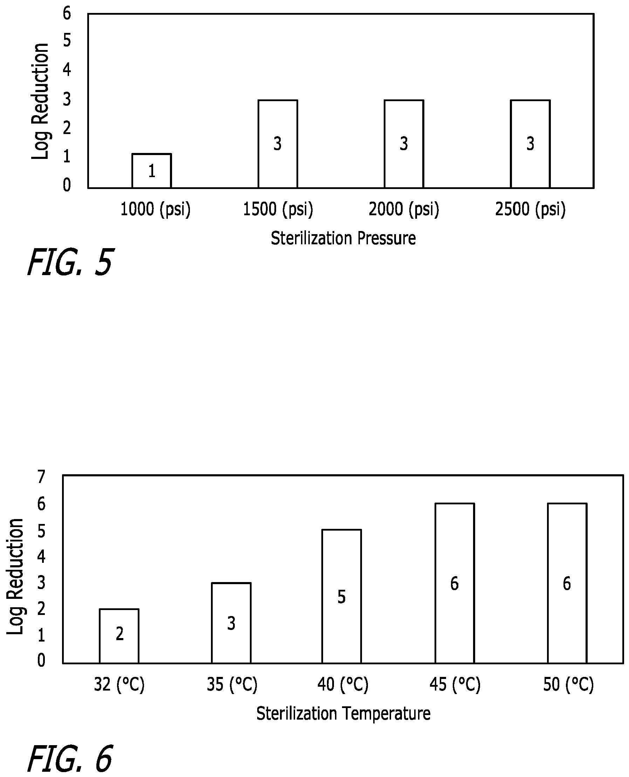

Pressure: To evaluate the sterilization pressure on sCO₂ sterilization efficacy, the bioindicator strips were sterilized under four different sCO₂ sterilization pressures, while keeping the sterilization temperature at 35 °C and the cycle duration at 90 minutes for all four sterilization pressures. The results are shown in FIG. 5, which shows that under the given conditions, the increase of sterilization pressure from 1000 psi to 1500 psi improves the sterilization efficacy from a one log microbial reduction to a three log microbial reduction, increasing the pressure beyond 1500 psi does not improve the sterilization efficacy.

Temperature: To evaluate the effect of processing temperature on the sCO₂ sterilization efficacy, the bioindicator strips were sterilized under five different sterilization temperatures while keeping the sCO₂ sterilization processing pressure fixed at 1500 psi and the cycle duration fixed at 90 minutes for each of the five sterilization temperatures. The results are shown in FIG. 6, which shows that as the sterilization temperature is increased from 32 °C to 45 °C, the sterilization efficacy increases from a 2 log reduction to a 6 log reduction but increasing the temperature to 50 °C does not further increase the sterilization efficacy.

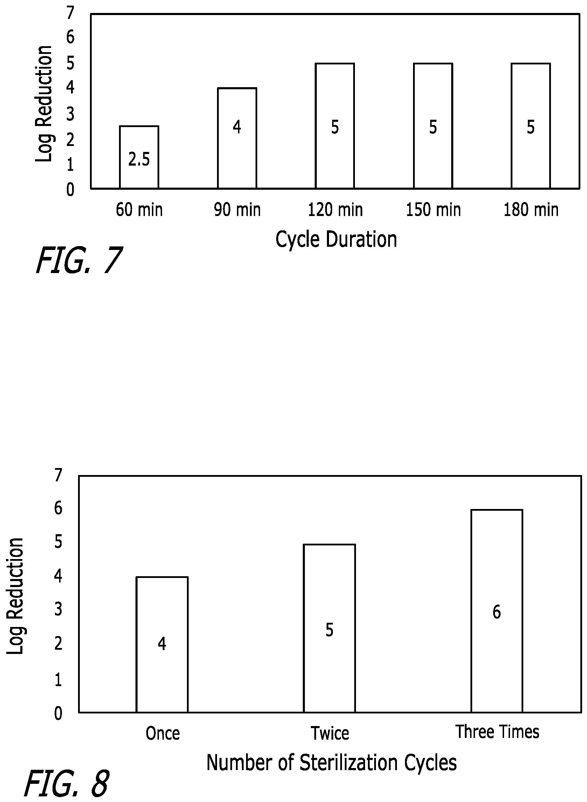

Sterilization cycle duration: To evaluate the effect of sterilization cycle duration on sCO₂ sterilization efficacy, 5 different cycle durations were selected while keeping the sCO₂ sterilization pressure fixed at 1500 psi and the sterilization temperature fixed at 40 °C minutes for each cycle duration. The results are shown in FIG. 7, which shows that increasing the cycle duration from 60 minutes to 120 minutes improves sterilization efficacy as measured by the microbial log reduction but increasing the cycle duration beyond 120 minutes does not further improve sterilization efficacy.

Number of sterilization cycles: To evaluate the effect of varying the number of sterilization cycles on sCO₂ sterilization efficacy, the number of cycles were varied from 1-3 while keeping sCO₂ sterilization processing pressure fixed at 1500 psi, the duration for each cycle fixed at 90 min, and the sterilization temperature fixed at 40 °C. The results are shown in FIG. 8, which shows that a six log reduction under the given conditions can be achieved by increasing the number of sterilization cycles from 1 to 3.

Based on the foregoing, in one embodiment, sCO₂ sterilization is carried out using sCO₂ at a sterilization pressure of 1500 psi, at a sterilization temperature of 40 °C, and using three sterilization cycles each 90 minutes in duration. In another embodiment, sCO₂ sterilization is carried out using sCO₂ at a sterilization pressure of 1500 psi, at a sterilization temperature of 45 °C, and using one sterilization cycle 90 minutes in duration.

Once the biological tissue (prior to or after being formed into a bioprosthetic device) has been dried using the drying process set forth above, it is placed in a gas permeable pouch or jar (for example, a pouch or jar comprising Tyvek), which is then placed in a sterilization chamber or container. In one embodiment of the methods disclosed herein, supercritical CO₂ is then injected into the chamber or container. The chamber/container is maintained at a pressure between about 1100 - 3000 psi and a temperature between 30 - 55 °C for 60 - 180 minutes. Following that the pressure and temperature of the chamber/container are brought to atmospheric conditions. In some embodiments of the methods disclosed herein, the process of subjecting biological tissue to sCO₂ pressures between about 1100 - 3000 psi and temperatures between 30 - 55 °C for 60 - 180 minutes is repeated 1 - 5 times.

Following sterilization with sCO₂ as set forth above, the biological tissue (or the bioprosthetic device comprising the biological tissue) is placed in a gas impermeable container (for example, a pouch or jar), ready to be prepared for delivery to end-users.

### Supercritical Carbon Dioxide Sterilization in the Presence of Hydrogen Peroxide

In alternative embodiments, sCO₂ sterilization is carried out in the presence of hydrogen peroxide (H₂O₂). The inventors have discovered that using H₂O₂ as an additive during sCO₂ sterilization, sCO₂ sterilization can achieve a six log microbial without damaging tissue mechanical properties. Thus, when using H₂O₂ as an additive during sCO₂ sterilization even at concentrations of 35%, there are minimal changes in tissue cross linking as measured by DSC (differential scanning calorimetry) and tissue strength as measured by UTS (uniaxial tensile strength) testing. In various embodiments aqueous H₂O₂ with an H₂O₂ concentration of 3% to 35% by weight is used as an additive during sCO₂ sterilization. In an exemplary embodiment, per one liter volume of the sCO₂ sterilization chamber, 0.1 ml to 10 ml of aqueous H₂O₂ with an H₂O₂ concentration of 3% to 35% by weight is used as an additive during sCO₂ sterilization in various embodiments of sCO₂ sterilization as set forth in the prior section above.

### Packaging

Following sterilization with sCO₂ as set forth above, the biological tissue (or the bioprosthetic device comprising the biological tissue) is placed in a gas impermeable container (for example, a pouch or jar), ready to be prepared for delivery to end-users.

### EXAMPLE 1: SURGICAL PATCH

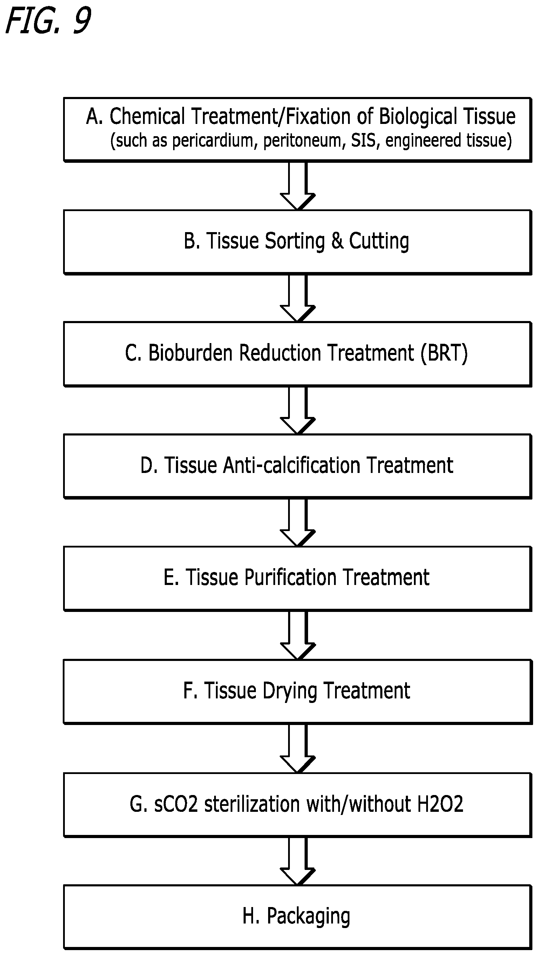

The overall flow chart of an exemplary embodiment for preparing a surgical patch according to the processes disclosed herein is shown in FIG. 9. In Step A, biological tissue, e.g., pericardium, peritoneum, SIS, or engineered tissue, is chemically treated/fixed using glutaraldehyde as set forth above in the section entitled "Fixation of Biological Tissue.". In Step B the chemically treated/fixed biological tissue is sorted to select appropriate tissue for surgical patches and one or more surgical patches are then cut from the sorted tissue. In Step C, the one or more surgical patches undergo a first bioburden reduction treatment as set forth above in the section entitled "Bioburden Reduction Treatment." In Step D, the one or more biological tissue surgical patches undergo anti-calcification treatment as set forth above in the section entitled "Anti-Calcification Treatment." In Step E, the one or more biological tissue surgical patches undergo tissue purification treatment as set forth in the section entitled "Tissue Purification Treatment." In step F, the one or more biological tissue surgical patches undergo tissue drying treatment (for example, glycerolization) as set forth above in the section entitled "Tissue Drying Treatment." In Step G, the one or more biological tissue surgical patches are placed in a gas permeable pouch or jar comprising, for example, Tyvek, and the one or more biological tissue surgical patches inside the gas permeable pouch or jar are subjected to sCO2 sterilization as set forth above in the section entitled "Supercritical Carbon Dioxide Sterilization." As an alternative, in some embodiments of Step G, the one or more biological tissue surgical patches inside the gas permeable pouch or jar are subjected to sCO2 sterilization in the presence of hydrogen peroxide as set forth above in the section entitled "Supercritical Carbon Dioxide Sterilization in the Presence of Hydrogen Peroxide." In Step H, the one or more sterilized biological tissue surgical patches are placed in gas impermeable container(s) (for example, a pouch or a jar), ready for packaging and shipping.

In various embodiments, the sequence of steps illustrated in FIG. 9 can be varied. Thus, for example, Step B can be performed after Step E. In other words, the biological tissue can be chemically treated/fixed, undergo bioburden reduction treatment, tissue anti-calcification treatment and tissue purification treatment, before it is sorted and cut into surgical patches. All such variations are contemplated withing the scope of the invention disclosed herein.

In Step A of an exemplary embodiment, bovine pericardial tissue obtained from the pericardial sac of a cow was immersed in a solution of 0.5% by weight glutaraldehyde buffered to a pH of approximately 7.4 by a suitable buffer (such as a phosphate buffer), for up to 1 day at ambient temperature. The 0.5% glutaraldehyde solution further comprised a mixture of surfactant (e.g., Tween^{®} 80) ethanol, and/or formaldehyde.

In Step B of the exemplary embodiment, the chemically treated/ fixed bovine pericardial tissue was sorted to select appropriate tissue for surgical patches and one or more surgical patches were then cut from the sorted tissue.

In Step C of the exemplary embodiment, the one or more surgical patches were rinsed in an ethanol/saline solution (less than 70% ethanol) to remove any excess chemicals adhering to the patches and, in a first bioburden reduction step, were contacted with a first bioburden reduction mixture containing i) a crosslinking agent (such as glutaraldehyde or formaldehyde) ii) a denaturing agent, (such as ethanol or isopropanol ) and iii) a surfactant (such as commercially available Tween^{®} 80 surfactant) with the following composition: glutaraldehyde 2± 0.2% by weight, ethanol 25.0±2.5% by weight, and Tween^{®} 80 1.2±0.2% by weight. The one or more surgical patches were immersed in this mixture for about 2 hours. During this immersion period the mixture was maintained at a temperature of 40±1 °C.

In Step D of the exemplary embodiment, the one or more surgical patches were rinsed in an ethanol/saline solution (less than 70% ethanol) to remove any excess bioburden reduction mixture adhering to the surgical patch(es). The one or more surgical patches were then contacted with normal saline to remove residual glutaraldehyde and other chemicals, and were then contacted with a 0.1 M glycine solution in phosphate buffered saline at a pH of 7.35 to 7.45.

In Step E of the exemplary embodiment, the one or more surgical patches were rinsed in an ethanol/saline solution (less than 70% ethanol) to remove any excess bioburden reduction mixture adhering to the tissue, and the surgical patch(es) were subjected to a tissue purification treatment comprising first immersing the surgical patch(es) in a 20 weight percent ethanol aqueous ethanol solution at room temperature for 60 minutes, and transferring the surgical patch(es) soaked in ethanol solution quickly to a chamber connected to a source of supercritical carbon dioxide (sCO₂). Supercritical CO₂ at a pressure of 2000 psi and a temperature of 45 °C was then injected into the chamber and the chamber maintained under supercritical conditions for 120 minutes. Carbon dioxide was then vented from the chamber and the purified biological tissue surgical patch(es) were removed from the chamber.

In Step F of the exemplary embodiment, the one or more biological tissue surgical patches were dried by contacting them with an-aqueous solution containing 80% by weight glycerol, 19% by weight ethanol, and 1%water for at least 2 hours at room temperature. After contact for 2 hours, the one or more biological tissue surgical patches were removed from the glycerol/ethanol solution and exposed to ambient air, at ambient room temperature and humidity. Typical conditions were ambient temperature of about 25 °C, and relative humidity less than about 50%. This exposure was performed in a clean room at ambient room conditions for up to 24 hours. This exposure allows the excess glycerol/ethanol mixture to drip off/evaporate from the biological tissue surgical patch(es), resulting in sufficiently dry biological tissue surgical patch(es) substantially devoid of any free water molecules. Sufficiently dry herein means that the if a sufficiently dry biological tissue surgical patch is subjected to sCO₂ sterilization, the biological tissue surgical patch will not freeze and irreversibly dehydrate.

In one alternate of Step G of the exemplary embodiment, the one or more dry biological tissue surgical patches were placed in a gas permeable pouch comprising Tyvek, which was then placed in a sterilization chamber. Supercritical CO₂ was then injected into the chamber and the chamber was maintained at a pressure 2000 psi and a temperature of 45 °C for 2 hours. Following that the pressure and temperature of the chamber were brought to ambient conditions. Supercritical CO₂ was then again injected into the chamber and the chamber was maintained at a pressure 2000 psi and a temperature of 45 °C for 2 hours. Following that the pressure and temperature of the chamber were brought back to ambient conditions. As an alternative, in some embodiments of Step G of the exemplary embodiment, per one liter volume of the sCO₂ sterilization chamber, 5 ml of aqueous H₂O₂ with an H₂O₂ concentration of 10% by weight is used as an additive during sCO₂ sterilization set forth in this paragraph above. A six log reduction in bacterial load of the surgical patches was obtained.

In Step H of the exemplary embodiment, the one or more biological tissue patches were then placed in a gas impermeable pouch, ready to be prepared for delivery to end-users.

### EXAMPLE 2: BIOPROSTHETIC HEART VALVE

Bioprosthetic heart valves comprising biological tissue are used to replace damaged or diseased native heart valves of patients. In vertebrate animals, the heart is a hollow muscular organ having four pumping chambers: the left and right atria and the left and right ventricles, each provided with its own one-way valve. The native heart valves are identified as the aortic, mitral (or bicuspid), tricuspid and pulmonary valves. Bioprosthetic heart valves comprising biological tissue can be used to replace any of these naturally occurring valves. A stented biological tissue bioprosthetic heart valve is constructed with biological-tissue valve leaflets which function much like leaflets of a natural human heart valve, imitating the natural action of the flexible native heart valve leaflets which seal against each other or coapt between adjacent tissue junctions known as commissures. Such bioprosthetic heart valves have been in use for decades and the details of the manufacture and construction of such valves are well known to those of ordinary skill in the art, for example, details disclosed in US patent 5,928,281, the entire disclosure of which is incorporated herein by reference.

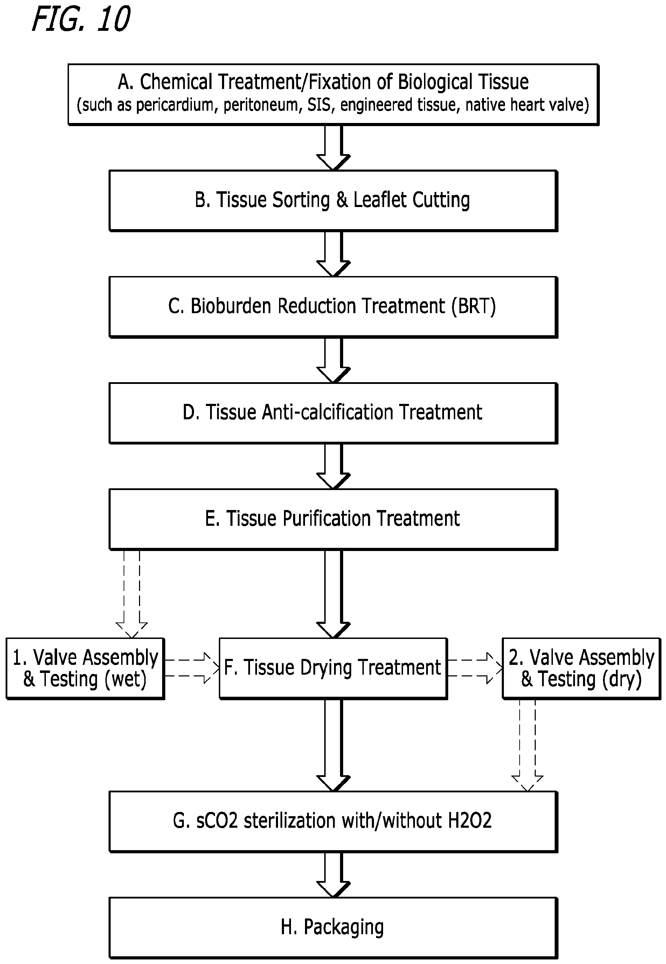

The overall flow chart of an exemplary embodiment for preparing a heart valve comprising biological tissue according to the processes disclosed herein is shown in FIG. 10. In Step A, biological tissue, e.g., pericardium, peritoneum, SIS, or engineered tissue, is chemically treated/fixed using glutaraldehyde as set forth above in the section entitled "Fixation of Biological Tissue." In an exemplary embodiment, bovine pericardial tissue obtained from the pericardial sac of a cow was immersed in a solution of 0.5% by weight glutaraldehyde buffered to a pH of approximately 7.4 by a suitable buffer (such as a phosphate buffer), for 1 day at ambient temperature. The 0.5% glutaraldehyde solution further comprises a mixture of surfactant (e.g., Tween^{®} 80), ethanol, and/or formaldehyde.

In Step B of the exemplary embodiment, chemically treated/fixed biological tissue is sorted to select appropriate tissue from which heart valve leaflets can be cut and heart valve leaflets are cut out of the selected tissue. The size and number of leaflets varies according to the intended application for the bioprosthetic heart valve.

In Step C, heart valve leaflets obtained from the chemically treated/fixed biological tissue undergo a first bioburden reduction treatment as set forth above in the section entitled "Bioburden Reduction Treatment." In Step C of the exemplary embodiment, the chemically fixed tissue leaflets were rinsed in an ethanol/saline solution (30% ethanol) to remove any excess chemicals adhering to the tissue leaflets and in a first bioburden reduction step were contacted with a first bioburden reduction mixture containing i) a crosslinking agent (such as formaldehyde) ii) a denaturing agent, (such as ethanol) and iii) a surfactant (such as commercially available Tween^{®} 80 surfactant) with the following composition: glutaraldehyde 2.0±0.2% by weight, ethanol 25.0±2.5% by weight, and Tween^{®} 80 1.2±0.2% by weight. The tissue leaflets were immersed in this mixture for about 16 hours. During this immersion period the mixture was maintained at a temperature of 40 °C.

In Step D, the biological tissue leaflets undergo anti-calcification treatment as set forth above in the section entitled "Anti-Calcification Treatment." In Step D of the exemplary embodiment, after the first bioburden reduction step, the fixed biological tissue leaflets were rinsed in an ethanol/saline solution (30% ethanol) to remove any excess bioburden reduction mixture adhering to the tissue leaflets. The fixed mammalian tissue leaflets were then contacted with normal saline to remove residual glutaraldehyde and other chemicals followed by capping the free aldehyde group with an amino acid solution, which in the exemplary embodiment was 0.1 M glycine solution in phosphate buffered saline at pH 7.35 to 7.45.

In Step E, the biological tissue leaflets undergo tissue purification treatment as set forth in the section entitled "Tissue Purification Treatment." In Step E of the exemplary embodiment, the biological tissue leaflets were rinsed in an ethanol/saline solution (less than 70% ethanol) and the biological tissue leaflets were subjected to a tissue purification treatment comprising first immersing the biological tissue leaflets in a 20 weight percent ethanol aqueous ethanol solution at room temperature for 60 minutes, and transferring the biological tissue leaflets soaked in ethanol solution quickly to a chamber connected to a source of supercritical carbon dioxide (sCO₂). Supercritical CO₂ at a pressure of 2000 psi and a temperature of 45 °C was then injected into the chamber and the chamber maintained under supercritical conditions for 120 minutes. Carbon dioxide was then vented from the chamber and the purified biological tissue leaflets removed from the chamber.

In step F, the biological tissue leaflets undergo tissue drying treatment (for example, glycerolization) as set forth above in the section entitled "Tissue Drying Treatment." The tissue drying treatment, in alternative embodiments, can be performed after or prior to assembling the biological tissue leaflets along with other structural elements into a bioprosthetic heart valve. In one alternate of Step F of the exemplary embodiment, the biological tissue leaflets along with other structural elements were assembled into a bioprosthetic heart valve and the assembled bioprosthetic heart valve was dried by contacting it with 70% ethanol for about 30 min, then by contacting with an aqueous solution containing 80% by weight glycerol, 19% by weight ethanol, and 1%water for at least 2 hours at room temperature. After contact for 2 hours, the bioprosthetic heart valve was removed from the glycerol/ethanol solution and exposed to ambient air, at ambient room temperature and humidity. Typical conditions were ambient temperature of about 25 °C, and relative humidity less than about 50%. This exposure was performed in a clean room at ambient room conditions for about 24 hours. This exposure allows the excess glycerol/ethanol mixture to drip off/evaporate from the heart valve, resulting in bioprosthetic heart valve with sufficiently dry tissue leaflets that are substantially devoid of free water. Sufficiently dry herein means that the if sufficiently dry soft mammalian tissue leaflets (or device comprising same) is subjected to sCO₂ sterilization, the biological tissue leaflets will not freeze and irreversibly dehydrate.

In an alternative Step F of the exemplary embodiment, the biological tissue leaflets were dried by contacting them with 70% ethanol for about 30 min, then by contacting with an aqueous solution containing 80% by weight glycerol, 19% by weight ethanol, and 1%water for at least 2 hours at room temperature. After contact for 2 hours, the biological tissue leaflets were removed from the glycerol/ethanol solution and exposed to ambient air, at ambient room temperature and humidity. Typical conditions were ambient temperature of about 25 °C, and relative humidity less than about 50%. This exposure was performed in a clean room at ambient room conditions for about 24 hours. This exposure allows the excess glycerol/ethanol mixture to drip off/evaporate from the leaflets, resulting in sufficiently dry tissue leaflets that are substantially devoid of free water. Sufficiently dry herein means that the if sufficiently tissue leaflets are subjected to sCO₂ sterilization, the biological tissue leaflets will not freeze and irreversibly dehydrate. The dry biological tissue leaflets along with other structural elements were then assembled into a bioprosthetic heart valve

The bioprosthetic valve is placed in a gas permeable pouch or jar comprising, for example, Tyvek. In Step G, the bioprosthetic valve in the gas permeable pouch or jar is subjected to sCO₂ sterilization as set forth above in the section entitled "Supercritical Carbon Dioxide Sterilization." As an alternative, in some embodiment of Step G, the biological tissue in the gas permeable pouch or jar is subjected to sCO₂ sterilization in the presence of hydrogen peroxide as set forth above in the section entitled "Supercritical Carbon Dioxide Sterilization in the Presence of Hydrogen Peroxide." Thus, in one alternative of Step G of the exemplary embodiment, the bioprosthetic heart valve inside a gas permeable pouch comprising Tyvek was placed in a sterilization chamber. Supercritical CO₂ was then injected into the chamber and the chamber was maintained at a pressure 2000 psi and a temperature of 45 °C for 2 hours. Following that, the pressure and temperature of the chamber were brought to ambient conditions. Supercritical CO₂ was then again injected into the chamber and the chamber was maintained at a pressure 2000 psi and a temperature of 45 °C for 2 hours. Following that, the pressure and temperature of the chamber were brought back to ambient conditions. In another alternative of Step G of the exemplary embodiment, per one liter volume of the sCO₂ sterilization chamber, 5 ml of aqueous HzOz with an H₂O₂ concentration of 10% by weight was used as an additive during sCO₂ sterilization set forth in this paragraph above. A six log reduction in bacterial load of the tissue leaflets of the bioprosthetic heart valve was obtained.

In Step H, the sterilized biological tissue is placed in a gas impermeable container (for example, a pouch or a jar), ready for packaging and shipping. In the exemplary embodiment, the bioprosthetic heart valve was placed in a gas impermeable pouch, ready to be prepared for delivery to end-users.

In various embodiments, the sequence of steps illustrated in FIG. 10 can be varied. Thus, for example, Step B can be performed after Step E. In other words, the biological tissue can be chemically treated/fixed, undergo bioburden reduction treatment, tissue anti-calcification treatment and tissue purification treatment, before it is sorted and cut into heart valve leaflets. Similarly, the heart valve leaflets can be assembled into a heart valve prior to tissue drying treatment, or alternatively, the heart valve leaflets can undergo tissue drying treatment prior to being assembled into a heart valve. All such variations are contemplated withing the scope if the invention disclosed herein.

Alternatively, the bioprosthetic heart valve prepared as above was sutured to a conduit graft that was impregnated with bioresorbable materials which in one embodiment was collagen, and in another embodiment was gelatin. In another embodiment, instead of suturing the bioprosthetic valve to the conduit graft, it was attached to it using a snap fit type connection.

Various alternative embodiments of the technology described herein are also contemplated. For example, surgical tissue patches and bioprosthetic heart valves can be prepared by altering the sequence of treatments steps outlined above. Further, instead of a stented bioprosthetic heart valve comprising bovine pericardium leaflets, a whole porcine valve can be prepared as a bioprosthetic device using the methods set forth above. Thus, a whole porcine valve harvested from a pig cadaver is subjected to the following treatment steps: (a) chemical fixation, (b) a first bioburden reduction treatment, (c) capping treatment, (d) a second bioburden reduction treatment, (e) drying treatment, (f) sterilization with sCO₂, and (g) placing the valve in a gas impermeable package. In another embodiment, following sterilization with sCO₂, the whole porcine valve is attached (either by suturing or a snap fit type connection) to a conduit graft that is impregnated with bioresorbable materials which in one embodiment is collagen, and in another embodiment is gelatin. The conduit valve is then placed in a gas impermeable package.

The above detailed descriptions of embodiments of the technology are not intended to be exhaustive or to limit the technology to the precise form disclosed above. Although specific embodiments of, and examples for, the technology are described above for illustrative purposes, various equivalent modifications are possible within the scope of the technology as those skilled in the relevant art will recognize. For example, while steps are presented in a given order, alternative embodiments may perform steps in a different order, and further may comprise fewer than the steps disclosed herein. The various embodiments described herein may also be combined to provide further embodiments.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms may also include the plural or singular term, respectively.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology. Further, while advantages associated with certain embodiments of the technology have been described in the context of those embodiments, other embodiments may also exhibit such advantages, and not all embodiments need necessarily exhibit such advantages to fall within the scope of the technology. Accordingly, the disclosure and associated technology can encompass other embodiments not expressly shown or described herein.

Terms and phrases used in this document, and variations thereof, unless otherwise expressly stated, should be construed as open ended as opposed to limiting. As examples of the foregoing: the term "including" should be read as mean "including, without limitation" or the like; the term "example" is used to provide exemplary instances of the item in discussion, not an exhaustive or limiting list thereof; and adjectives such as "conventional," "traditional," "normal," "standard," "known" and terms of similar meaning should not be construed as limiting the item described to a given time period or to an item available as of a given time, but instead should be read to encompass conventional, traditional, normal, or standard technologies that may be available or known now or at any time in the future. Likewise, a group of items linked with the conjunction "and" should not be read as requiring that each and every one of those items be present in the grouping, but rather should be read as "and/or" unless expressly stated otherwise. Similarly, a group of items linked with the conjunction "or" should not be read as requiring mutual exclusivity among that group, but rather should also be read as "and/or" unless expressly stated otherwise. Furthermore, although items, elements or components of the disclosure may be described or claimed in the singular, the plural is contemplated to be within the scope thereof unless limitation to the singular is explicitly stated. The presence of broadening words and phrases such as "one or more," "at least," "but not limited to" or other like phrases in some instances shall not be read to mean that the narrower case is intended or required in instances where such broadening phrases may be absent.

## Claims

1. A method of sterilizing a piece of soft mammalian tissue harvested from an animal cadaver, the method comprising:
immersing the piece of soft mammalian tissue in a chemical fixative solution;
contacting the piece of soft mammalian tissue with a first bioburden reduction mixture;
contacting the piece of soft mammalian tissue with a capping solution;
subjecting the piece of soft mammalian tissue to a tissue purification process;
contacting the piece of soft mammalian tissue with a tissue drying mixture; and
contacting the piece of soft mammalian tissue with supercritical carbon dioxide.

2. The method of claim 1, wherein the step of immersing the piece of soft mammalian tissue in a chemical fixative solution comprises:
immersing the piece of soft mammalian tissue in an aqueous glutaraldehyde solution comprising less than 10% glutaraldehyde by weight, a surfactant, ethanol and formaldehyde, wherein the piece of soft mammalian tissue is immersed in said aqueous glutaraldehyde solution for a period of less than14 days.

3. The method of claim 2, wherein the aqueous glutaraldehyde solution comprises 0.5% glutaraldehyde by weight and the pH of the aqueous glutaraldehyde solution is greater than 7, and wherein the piece of soft mammalian tissue is immersed in said aqueous glutaraldehyde solution for one day.

4. The method of any preceding claim, wherein the first bioburden reduction mixture comprises less than 5% by weight glutaraldehyde, less than 25% by weight ethanol and less than 2% by weight Tween 80, wherein the piece of soft mammalian tissue is contacted with the first bioburden reduction mixture for a time period of less than 7 days while the temperature of the first bioburden reduction mixture is maintained at less than 50 °C.

5. The method of claim 4, wherein the piece of soft mammalian tissue is contacted with the first bioburden reduction mixture for a time period of 16 hours while the temperature of the first bioburden reduction mixture is maintained at about 40 °C.

6. The method of any preceding claim, wherein the capping solution comprises 0.1M glycine in sufficient phosphate buffer to maintain the pH of the capping solution above 7.0,
wherein the piece of soft mammalian tissue is contacted with the capping solution for about 4 hours while maintaining the temperature of the capping solution at about 30 °C.

7. The method of any preceding claim, wherein the tissue purification process comprises:
immersing the soft mammalian tissue in an aqueous ethanol solution comprising at least 5% by weight ethanol for a period of greater than one minute;
transferring the soft mammalian tissue from the aqueous ethanol solution to a chamber; and
exposing the soft mammalian tissue in the chamber to supercritical carbon dioxide.

8. The method of claim 7, wherein the piece of soft mammalian tissue is exposed to supercritical carbon dioxide at a pressure greater than about 1100 psi and a temperature greater than about 31.1 °C.

9. The method of any preceding claim, wherein the tissue drying mixture comprises an aqueous mixture comprising less than 95% by weight glycerol, less than 70% by weight ethanol, and less than 5% by weight water,
wherein the piece of soft mammalian tissue is contacted with the tissue drying mixture for a time period of less than 24 hours and at a temperature of about 30 °C.

10. The method of any preceding claim, wherein the step of contacting the soft mammalian tissue with supercritical carbon dioxide comprises:
placing the piece of soft mammalian tissue in a gas permeable pouch;
placing the gas permeable pouch containing the piece of soft mammalian tissue in a sterilization chamber; and
injecting supercritical carbon dioxide into the sterilization chamber,
wherein the gas permeable pouch containing the piece of soft mammalian tissue is kept in the sterilization chamber for a time period of between 60 - 180 minutes while the sterilization chamber containing supercritical carbon dioxide is maintained at a pressure between about 1100 - 3000 psi, and a temperature between about 31 - 55 °C.

11. The method of any preceding claim, wherein the step of contacting the soft mammalian tissue with supercritical carbon dioxide comprises:
placing the piece of soft mammalian tissue in a gas permeable pouch;
placing the gas permeable pouch containing the piece of soft mammalian tissue in a sterilization chamber, wherein the sterilization chamber contains an aqueous solution of hydrogen peroxide having a concentration of 5% by weight hydrogen peroxide, wherein the amount of aqueous hydrogen peroxide is 5 ml per liter of the volume of the sterilization chamber; and
injecting supercritical carbon dioxide into the sterilization chamber,
wherein the gas permeable pouch containing the piece of soft mammalian tissue is kept in the sterilization chamber for a time period of between 60 - 180 minutes while the sterilization chamber containing supercritical carbon dioxide is maintained at a pressure between about 1100 - 3000 psi, and a temperature between about 31 - 55 °C.

12. A method of sterilizing a piece of soft mammalian tissue harvested from an animal cadaver, the method comprising:
immersing the piece of soft mammalian tissue in an aqueous glutaraldehyde solution comprising less than 10% glutaraldehyde by weight, a surfactant, ethanol and formaldehyde, wherein the piece of soft mammalian tissue is immersed in said aqueous glutaraldehyde solution for a period of less than14 days;
contacting the piece of soft mammalian tissue with a bioburden reduction mixture comprising less than 5% by weight formaldehyde, less than 25% by weight ethanol and less than 2% by weight Tween 80, wherein the piece of soft mammalian tissue is contacted with the bioburden reduction mixture for a time period of less than 7 days while the temperature of the bioburden reduction mixture is maintained at less than 50 °C;
contacting the piece of soft mammalian tissue with a tissue drying mixture comprising an aqueous mixture comprising less than 95% by weight glycerol, less than 70% by weight ethanol, and less than 5% by weight water, wherein the piece of soft mammalian tissue is contacted with the tissue drying mixture for a time period of less than 24 hours and at a temperature of about 30 °C;
placing the piece of soft mammalian tissue in a gas permeable pouch;
placing the gas permeable pouch containing the piece of soft mammalian tissue in a sterilization chamber; and
injecting supercritical carbon dioxide into the sterilization chamber,
wherein the gas permeable pouch containing the piece of soft mammalian tissue is kept in the sterilization chamber for a time period of between 60 - 180 minutes while the sterilization chamber containing supercritical carbon dioxide is maintained at a pressure between about 1100 - 3000 psi, and a temperature between about 31 - 55 °C.

13. The method of claim 12, wherein prior to the step of contacting the piece of soft mammalian tissue with the tissue drying mixture, the piece of soft mammalian tissue undergoes anti-calcification treatment comprising:
washing the soft mammalian tissue with normal saline; and
contacting the soft mammalian with a solution comprising 0.1 M glycine and phosphate buffered saline at pH of 7.35 to 7.45.

14. The method of claim 13, wherein after the step of anti-calcification treatment, the piece of soft mammalian tissue undergoes tissue purification treatment comprising:
immersing the soft mammalian tissue in an aqueous ethanol solution comprising at least 5% by weight ethanol for a period of greater than one minute;
transferring the soft mammalian tissue from the aqueous ethanol solution to a chamber; and
exposing the soft mammalian tissue in the chamber to supercritical carbon dioxide.

15. A method of sterilizing a piece of soft mammalian tissue harvested from an animal cadaver, the method comprising:
contacting the piece of soft mammalian tissue with a tissue drying mixture comprising an-aqueous mixture comprising less than 95% by weight glycerol, less than 70% by weight ethanol, and less than 5% by weight water, wherein the piece of soft mammalian tissue is contacted with the tissue drying mixture for a time period of less than 24 hours and at a temperature of about 30 °C;
placing the piece of soft mammalian tissue in a gas permeable pouch;
placing the gas permeable pouch containing the piece of soft mammalian tissue in a sterilization chamber; and
injecting supercritical carbon dioxide into the sterilization chamber,
wherein the gas permeable pouch containing the piece of soft mammalian tissue is kept in the sterilization chamber for a time period of between 60 - 180 minutes while the sterilization chamber containing supercritical carbon dioxide is maintained at a pressure between about 1100 - 3000 psi, and a temperature between about 31- 55 °C.

16. A method of manufacturing a tissue surgical patch comprising a piece of soft mammalian tissue harvested from an animal cadaver, the method comprising:
contacting the piece of soft mammalian tissue with a tissue drying mixture comprising an aqueous mixture comprising less than 95% by weight glycerol, less than 70% by weight ethanol, and less than 5% by weight of water, wherein the piece of soft mammalian tissue is contacted with the tissue drying mixture for a time period of less than 24 hours and at a temperature of about 30 °C;
cutting the tissue surgical patch out of the piece of soft mammalian tissue;
placing the tissue surgical patch in a gas permeable pouch;
placing the gas permeable pouch containing the tissue surgical patch in a sterilization chamber; and
injecting supercritical carbon dioxide into the sterilization chamber,
wherein the gas permeable pouch containing the tissue surgical patch is kept in the sterilization chamber for a time period of between 60 - 180 minutes while the sterilization chamber containing supercritical carbon dioxide is maintained at a pressure between about 1100 - 3000 psi, and a temperature between about 31 - 55 °C.
